Europäisches Patentamt

(19) European Patent Office

Office européen des brevets

(11) Publication number: **0 007 157**
**A2**

# EUROPEAN PATENT APPLICATION

(21) Application number: **79300738.6**

(22) Date of filing: **01.05.79**

(51) Int. Cl.³: **A 61 F 11/04**

(30) Priority: **04.05.78 AU 4285/78**

(43) Date of publication of application:
**23.01.80 Bulletin 80/2**

(84) Designated Contracting States:
**DE FR GB IT NL**

(71) Applicant: **THE UNIVERSITY OF MELBOURNE**
**Grattan Street**
**Parkville Victoria(AU)**

(72) Inventor: **Clark, Graeme Milbourne**
**"Karuah" 13 Bannon Road**
**Eltham, Victoria(AU)**

(72) Inventor: **Patrick, James Finlay**
**13 O'Shanassy Street**
**North Melbourne, Victoria(AU)**

(72) Inventor: **Bailey, Quentin Richard**
**2 Cloverlea Street**
**Blackburn, Victoria(AU)**

(74) Representative: **Hartley, David et al,**
**c/o Withers & Rogers 4 Dyer's Buildings Holborn**
**London, EC1N 2JT(GB)**

(54) Improved electrode array and method of making the same.

(57) An electrode array 10 specifically for implantation into the human cochlea comprising a biologically inert tube 20 about which is formed a number of electrodes 13 each of which is associated with an insulated conducting wire 14 which passes through a slot 16 in the tube 20 and at one end thereof; the electrodes each being formed of a strip of thin foil and lie substantially within the diameter of the tube 20, the inner end 12 of which is tapered for easy insertion into the cochlea and to minimise trauma during the insertion procedure.

EP 0 007 157 A2

Croydon Printing Company Ltd.

## IMPROVED ELECTRODE ARRAY

This invention relates to an improved electrode array and in particular to an array adapted for surgical implantation and specifically for implantation in the human cochlea.

The cochlea is a spiral bone in the temporal bone which contains the organ and nerves of hearing by which sound is perceived and, at the present time, in various countries of the world, attempts are being made to directly stimulate the auditory nerves in the cochlea so that a person suffering from nerve deafness can "hear".

In order to stimulate the nerves it is necessary to insert at least one electrode in the cochlea and this insertion can be done through the round window or by drilling a hole into the cochlea. We consider that it is desirable to stimulate the nerves at various spaced positions along the length of the cochlea as the different frequencies perceived by a person with normal hearing are developed by stimulation of various nerves along the cochlea. The locations of these nerves have a relationship with the frequency perceived and, thus, it is essential to use an electrode array with electrodes at the required spaced positions and which, in itself, can be passed along the spiral of the cochlea.

There have been previous forms of electrode arrays proposed, one of which was a stranded wire array in which electrodes were formed by the termination of the wires at various positions along the length of the array, the terminated wire being stripped and spirally wound around the other wires of the array. Such stranded electrodes provided the necessary electronic properties and, to a greater or lesser extent, the required mechanical properties. These electrodes were not satisfactory as it was difficult to form them with a smooth outer surface and on insertion they tended to cause trauma. There have also been proposed sputtered arrays but it has been difficult and expensive to attempt to manufacture such arrays as they need application of a very advanced form of technology.

It is an object of the invention to provide an electrode array which is sufficiently flexible to be able to be passed around the spiral of the cochlea whilst being sufficiently stiff to be fed and which has electrodes spaced at predetermined spacings which electrodes are adapted to contact the nerve endings in the cochlea.

The invention includes an electrode array comprising a flexible biologically inert tube, a number of electrodes being conducting bands located at predetermined spaced distances along portion of the length of the tube, the electrodes lying, generally, within the diameter of the tube and a conducting wire associated with each electrode and passing to the interior of the tube through a slot, aperture or the like in the tube at a position beneath the associated electrode to which the wire is connected and along the length of the tube to one end thereof.

If required the tube may be filled completely or in part with a material with appropriate mechanical properties.

Preferably each electrode is formed from a thin film of a biologically inert metal, such as platinum, and the tube can be of a biologically clean grade silicone rubber, such as that sold under the Trade Mark "Silastic".

In order that the invention may be more readily understood we shall describe one particular form of electrode array manufactured in accordance with the invention in relation to the accompanying drawings, in which:-

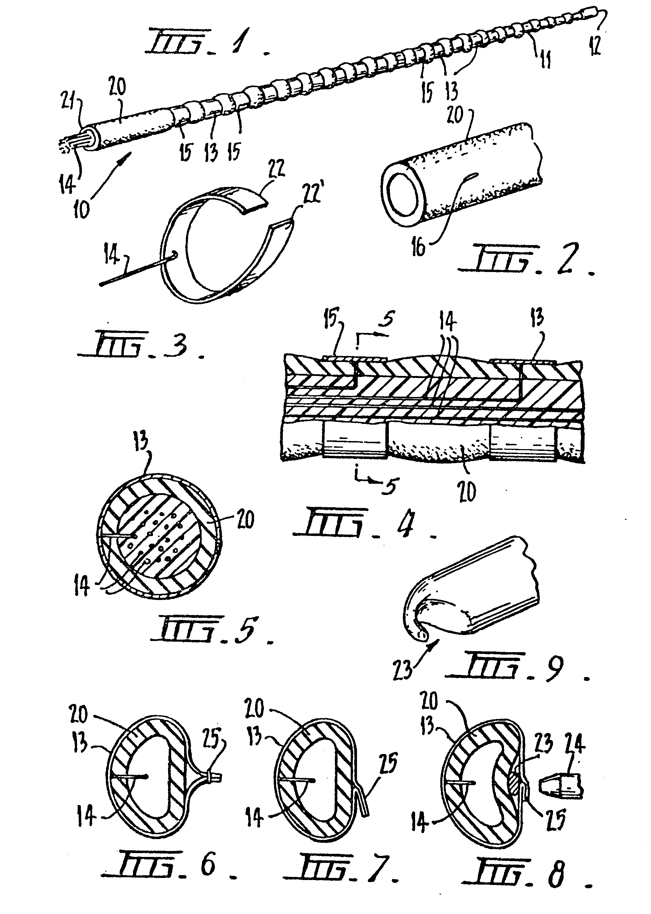

Fig. 1 is a part perspective view of an electrode array made in accordance with the invention showing all the electrodes but the portion of the array which does not have electrodes is truncated;

Fig. 2 is a perspective view of part of the tube about which the array is formed showing a slot through which a conductor can pass;

Fig. 3 shows a strip from which an electrode can be formed and a conductor attached thereto;

Fig. 4 is a partial sectional elevation of the array of the invention;

Fig. 5 is a section along line 5-5 of Fig. 4;

Figs. 6, 7 and 8 show the steps in the location of an electrode about the tube; and

Fig. 9 is a specially designed welding electrode for use in the operation of Fig. 8.

The array 10 is formed about a clean grade silicon rubber tube 20 which may be of "Silastic" and, as a matter of practice, we prefer to use a small diameter medical grade "Silastic" tube which has an external diameter of 560-640 microns. The internal diameter is approximately half this, say 320 microns. Preferably the inner (in the sense of being first introduced) portion 11 of the array, containing approximately one half of the total number of electrodes is tapered so the inner end 12 has a diameter approximately half of the diameter of the remainder of the tube. The inner end is formed to be smooth and rounded to minimise trauma on insertion. Thus, if the outer diameter of the main portion of the tube is 640 microns then the diameter of the inner end is of the order of 300 microns. The electrodes 13 are preferably of thin foil platinum and this preferably has a thickness of the order of 165 microns. We believe that the optimum area of each electrode, which is wrapped about the tube is of the order of $0.5mm^2$ and in order to achieve this area when the electrode is connected we use a film 2 mm long and 0.3 mm wide. The conducting wire 14 we use, again, is biologically inert and in order to achieve the required mechanical properties is 90% platinum

0007157

and 10% rhodium. The wire diameter is of the order of 25 microns and each individual wire is insulated with polytetra-fluoroethylene (PTFE). The silastic tube may be filled completely or in part with a medical grade silicone rubber.

The number of electrodes required depends on the associated equipment to be used and could, in an extremely simple case, comprise a single active electrode placed along the tube at a position where most successful stimulation is believed to occur. There could, alternatively, be a plurality of active electrodes 13 at various spacings and, if required, earth electrodes could be placed at each end of the array or, we believe preferably, at each end and between each pair of active electrodes. This arrangement permits the use of bi-polar stimulation, if required, and we believe that such stimulation can be the most desirable form. In the particular form of array illustrated we have ten active electrodes designated 13 and ten earth electrodes designated 15 although it will be appreciated that the electrodes are identical each being between a pair of active electrodes and at the inner end of the array.

The spacing of the active electrodes 13 is preferably 1.5 mm and with intermediate earth electrodes 15 the electrode spacing along the active portion of the array is 0.75 mm. This spacing can be reduced to provide more active electrodes in area required for speech formant frequencies.

In order to manufacture the array 10, we initially weld the conducting wires 14 to approximately the centre of what will be the underside of the electrodes 13. We then form in the "Silastic" tube at 0.75 mm spacings, slots 16 which have a length of less than the width of the electrode, that is less than 0.3 mm.

As each electrode 13 is formed in a similar manner, we shall now describe the location of a single electrode and it will be understood that the operation must be repeated, in the illustrated embodiment, some twenty times to complete the array.

The wire 14 is first fed into the slot 16 and along the tube 20 towards the outer end 21 thereof,

that is the end external to the cochlea when the array is
implanted.    The electrode, when the wire is fully fed, is
then wrapped around the tube and the two ends 22,22' are
grasped and pressure is applied until the tube 20 is deformed
inwardly by a distance slightly more than the thickness of
the film.    The ends, at this position, are then welded.
This is the condition shown in Fig. 6.    This leaves a tail
25 on the electrode and the tail is then deformed until
it lies adjacent portion of the electrode which is passed
around the tube.    This is the condition shown in Fig. 7.
At this time a welding electrode 23, which is an electrode
specifically designed for this purpose, is passed between
the electrode 13 and the tube 20 and a second electrode 24
is brought into contact with the tail which then becomes
welded to the remainder of the electrode, the arrangement
being such that even at the position where there are three
thicknesses of electrode foil, these will lie below the original
diameter of the tube.    The outer ends of each of the wires
can be terminated in any required way, normally by a connector
component.    The inner end of the electrode can also be sealed
in any required way, the requirement being, of course, that
it is smoothly finished as it must lead the array into the
cochlea in such a way as to cause minimal damage to the nerve
endings therein.    It is also for this reason that the inner
portion 11 is tapered.

                   To provide improved mechanical properties
for the electrode we prefer to inject into the tube, after
all the conducting wires 14 have been passed therethrough,
uncured silicone rubber which then cures to form a monolithic
structure.    This also acts as an additional insulant.

                   We have found that the electrode array
of the invention is extremely satisfactory as, to insert
the array, the necessary surgical treatment demands that
the assembly is deformed through a relatively great angle
and then, of necessity, is fed into the cochlea and around
the spiral therein by forceps external of the cochlea.
When the surgeon is inserting the array it is not necessarily
possible to avoid the electrodes with the forceps and the
electrodes of the invention are sufficiently flexible as
to be capable of accepting deformation and to be sufficiently

0007157

elastic to return substantially to their initial condition.

We have described herein one mechanical way of making the electrode array and the manufacture of the array in this way is quite satisfactory, although it demands a high degree of skill.

An alternative method of manufacture which can be used is to locate the completed electrode and wire assemblies in a die and to inject uncured silicone rubber into the die so that when cured there is an array in which the wires are fully encapsulated and the electrodes are flush with the surface. In this form the seam of the electrodes would be internal so that a smooth and flush outer surface is obtained.

Claims

1.        An electrode array comprising a flexible biologically inert tube 20, a number of electrodes 13 being conducting bands located at predetermined spaced distances along portion of the length of the tube, the electrodes lying, generally, within the diameter of the tube and a conducting wire 14 associated with each electrode and passing to the interior of the tube through a slot, aperture or the like 16 in the tube at a position beneath the associated electrode to which the wire is connected and along the length of the tube to one end thereof.

2.        An array as claimed in claim 1 wherein the tube 20 is of a flexible resiliant material.

3.        An array as claimed in claim 2 wherein the tube 20 is of silicone rubber.

4.        An array as claimed in any preceding claim wherein each electrode 13 is a metal foil having a conducting wire 14 located approximately centrally of its longitudinal axis, the electrode passing about the tube, the ends 22,22'being connected.

5.        An array as claimed in claim 4 wherein the electrode is formed from a rectangular foil which is placed about the tube and welded, the tail 25 then being folded against the remainder of the electrode and welded thereto.

6.        An array as claimed in any preceding claim wherein the tube is filled with a silicone rubber.

7.        An array as claimed in any preceding claim wherein the inner end 12 of the tube 20 is tapered.

8.        A method of forming an electrode array comprising forming slits or apertures 16 in a tube 20 of resilient flexible material, forming a number of electrode components 13 being rectangular strips of conducting material having a conducting wire 14 connected thereto, passing a conducting wire through each slit, feeding it to the end of the tube which is to be the outer end, wrapping the foil about the tube so the two free ends 22,22' form a tongue, welding these, folding the tongue against the adjacent portion of the conductor and welding the tongue to the adjacent portion.

0007157

9.          A method as claimed in claim 8 wherein the tube is filled with silicone rubber after the electrodes have been formed.

10.          A method as claimed in claim 8 or claim 9 wherein the end 12 of the tube 20 which is to be the inner end is formed with a taper.

11.          A method of forming an electrode array comprising locating cylindrical electrode elements having conducting wires affixed thereto into a die, leading these wires from one end of the die, injecting uncured silicone rubber into the die, which is basically cylindrical and after the silicone rubber has cured removing the array from the die.

0007157

FIG. 1.

FIG. 2.

FIG. 3.

FIG. 4.

FIG. 5.

FIG. 6.

FIG. 7.

FIG. 8.

FIG. 9.